# EUROPEAN PATENT APPLICATION

(11) **EP 2 745 703 A1**
(43) Date of publication of application: **25.06.2014**
(21) Application number: 13005855.5
(22) Date of filing: 17.12.2013
(51) Int. Cl.: A23K 1/14, A23K 1/16, A23K 1/18

(54) **Natural feed mixture for breeding domesticated water birds**

(30) Priority: 18.12.2012 HU 1200743
(71) Applicant: Libafood Tech Kft., 4032 Debrecen (HU)
(72) Inventor: Szilvássy, Zoltán, 4225 Debrecen-Józsa (HU); Tóth, Péter, 4025 Debrecen (HU); Földi, Enikö, 4030 Debrecen (HU); Lampé, Zsolt Tibor, 4027 Debrecen (HU); Mészáros, József, 4032 Debrecen (HU); Anton, Pál, 4032 Debrecen (HU)
(74) Representative: Emri, Jozsefné

(57) **Abstract**

The invention relates to a natural feed mixture for breeding domesticated water birds, comprising a high carbohydrate containing basic component poor in methyldonors, a component rich in carbohydrates, having hepatic insulin-sensitizing properties, hypothalamic appetite-stimulating plant parts, natural substances blocking cholesterol synthesis in the liver, and an additive for protecting the formation of non-cholesterolmevalonate products.

## Description

The invention relates to a natural feed mixture for breeding domesticated water birds, comprising a high carbohydrate containing basic component poor in methyl-donors, a component rich in carbohydrates, having hepatic insulin-sensitizing properties, hypothalamic appetite-stimulating plant parts, natural substances blocking cholesterol synthesis in the liver, and an additive for protecting the formation of non-cholesterol-mevalonate products.

Fattened liver, "foie gras" has been a valued and popular culinary delicacy for thousands of years. It sells well in the world market, and enjoys a consistently high demand. Fattened liver is produced by force-feeding predominantly domesticated water birds (geese, Mulard ducks). On the basis of CIFOG data, production is limited to only a few countries, France accounts for about 3/4th of the world production of fattened poultry liver, Hungary for about 10%, followed by Bulgaria with 7%. Force-feeding has been banned in most member states of the European Union, with the exception of France and Hungary. According to the legislation currently in force in Hungary, force-feeding in a humane manner is not prohibited. However, due to the strengthening of animal rights movements, force-feeding will be most probably banned in Hungary as well sooner or later.

In addition to the method of force-feeding, currently there is no other accepted technology for the production of fattened goose and duck liver. For liver production vigorous geese in good condition are selected, and undergo a preparatory feeding period. During this period the animals are acclimated to consuming large amounts of green, to be able to take up and digest large amounts of corn. The force-feeding of geese is started at the age of 8-9 weeks, but older geese can also be subjected to force-feeding after a 1-2 week preparatory period. In the preparatory period mass feed widening the food pipe plays a major role. For this purpose, mixing grated pumpkin or beet, possibly fallen fruit, chopped green feed to the feed can be considered.

Manual force-feeding is highly skilled, time-consuming and laborious work, replaced by mechanical force-feeding, where possible. However, mechanical force-feeding causes negative stress effects in the animals, and can also cause serious mechanical injuries to the food pipe. Therefore there have been several attempts at improving the technology of force-feeding with respect to both the composition of the feed used (Hungarian patent No. 158 330) and the force-feeding machine (Hungarian patent No. 155 704).

There have also been attempts at producing fattened goose liver without force-feeding. The genetic methods, because of the low population growth rate of the species, have yielded little result in practice. Hungarian patent No. 162 614 relates to a method for producing fattened goose liver biologically in such a way that the thyroid function of geese is artificially reduced or eliminated by various methods (laser, chemical agents, immuno-biological methods). Despite the effectiveness of the method, it is obviously not a solution to the problem.

The aim of the invention is to produce fattened goose liver without force-feeding, in a simple way, by a suitable choice of the composition of the feed.

The set aim was achieved on the basis of the pharmacological properties of natural feed components.

The invention is based on the recognition that by using MCH-1 receptor agonists, a significant and rapid increase in body weight can be achieved in vertebrate animals through appetite stimulation.

The aim of our invention, however, is not simply to achieve an increase in body weight, but also to achieve an increase predominantly in liver weight in the geese fed on the special feed, or feed supplement. Therefore active substances ensuring a gain predominantly in liver weight are also needed: a component rich in carbohydrates, having hepatic insulin-sensitizing properties, natural substances blocking cholesterol synthesis in the liver, and additives for protecting the formation of non-cholesterol-mevalonate products.

The invention relates to a feed mixture for breeding liver geese, comprising a high carbohydrate containing basic component poor in methyl-donors, optionally a non-carbohydrate-based component of high caloric value and/or a carbohydrate or protein, amino acid mixture, fat, as well as phospholipid, cholesterol ester and fatty acid-containing component, and natural flavouring as the main feed, and the main feed is supplemented with a component rich in carbohydrates, having hepatic insulin-sensitizing properties, as well as a feed supplement, comprising hypothalamic appetite-stimulating plant parts, natural substances blocking cholesterol synthesis in the liver, and an additive for protecting the formation of non-cholesterol-mevalonate products.

The invention also relates to a feed supplement, comprising hypothalamic appetite-stimulating plant parts, natural substances blocking cholesterol synthesis in the liver, and an additive for protecting the formation of non-cholesterol-mevalonate products.

It is an old experience that the best force-feeding material is corn. According to observations, from the point of view of liver production there are differences between different corn varieties, and the factors influencing the quality of corn, e.g. the soil, also affect the effectiveness of liver production. The use of corn as the main feed is justified by its very high carbohydrate content and that it has the lowest methionine and choline (methyl-donor) content among cereals.

When examining the physiological effects of natural substances, plant extracts and plant parts used as nutrients, it was found that fenugreek (Trigonella foenum-graecum) seed, and the seed juice pressed from it, when administered orally, by-presumably - activating the sensory fibres of the N. vagus ascending to the hypothalamus, causes indirect MCH-1 receptor excitement that can be suspended in rats by MCH-1 specific blockers. In view of the fact that the MCH-1 receptor appears to have an appetite-stimulating, or body weight-increasing effect in all vertebrates, the MCH-1 receptor stimulation is a suitable mechanism for achieving an increase in body weight in geese as well. Therefore fenugreek seed or seed juice can be preferably used as a hypothalamic appetite-stimulating component.

In addition to the indirect MCH-1 receptor agonist fenugreek seed, substances suitable for stimulating an increase in liver weight are also needed. From the substances suitable for this purpose, a preferably used component is the insulin-sensitizing sweet potato (caiapo), which according to studies exerts its effect predominantly through the insulin-dependent inhibition of hepatic glucose production.

Bile acids, preferably lithocholic acid and ursodeoxycholic acid, are also substances promoting an increase predominantly in liver weight, by affecting bile acid synthesis in the liver through the inhibition of 7α OH-ase, and by blocking cholesterol synthesis in the liver.

In addition, an additive for protecting the formation of non-cholesterol-mevalonate products is also needed. Most preferred for this purpose is farnesol oil (acyclic sesquiterpene alcohol), which is a component of several natural oils (e.g. lemongrass, orange blossom, musk, impatients).

The main feed can be supplemented with a non-carbohydrate-based component of high caloric value, e.g. pig fat, and/or a carbohydrate or protein, amino acid mixture, fat, as well as phospholipid, cholesterol ester, and fatty acid-containing component.

According to a preferred embodiment of the invention, the feed mixture comprises 50 - 90 w% of corn, 50 - 90 w% of sweet potato, 0.1 - 10 w% of fenugreek seed, 0.1 -10 w% of a mixture of lithocholic acid and ursodeoxycholic acid, 0.1 -1 w% of fanresol oil, 1 -10 w% of pig fat, and 0.1 - 2 w% of iodized NaCl.

The feed supplement according to the invention comprises preferably 1 - 99 w% of fenugreek seed, 0.1 - 10 w% of a 1:20/20:1 mixture of lithocholic acid and ursodeoxycholic acid, and 0.1 - 1 w% of farnesol oil.

The invention is described in more detail in the following example.

### Example:

The following "pilot" experiment was performed with the feed mixture according to the invention.

Fifteen 8-week-old grey Landes geese were used for the experiment. The geese were divided into 3 groups.
Group 1: received conventional goose feed;
Group 2: 5 animals were force-fed twice a day with the commonly used, salty, corn-based force-feeding material;
Group 3: and 5 animals received the feed mixture according to the invention.

Composition of the feed mixture:

| | |
|---|---|
| Corn | 60 w% |
| Sweet potato | 30 w% |
| Powdered pig fat | 3 w% |
| Iodized NaCl | 0.9 w% |
| Feed supplement | 10 w% (95 w% of fenugreek seed + 1 w% of a mixture of lithocholic acid and ursodeoxycholic acid + 0.1 w% of farnesol oil) |

| | |
|---|---|
| The results of the experiment are shown in Figure 1. Data: mean±S.D. in 5 animals per group. *: significant difference compared to normal feeding at p<0.05. | |

On the basis of the results, by feeding with the feed mixture according to the invention the same increase in liver weight can be achieved as by force-feeding (with conventional force-feeding feed).

The advantage of the invention is that it allows quality fattened liver production simply through feeding, with the elimination of force-feeding.

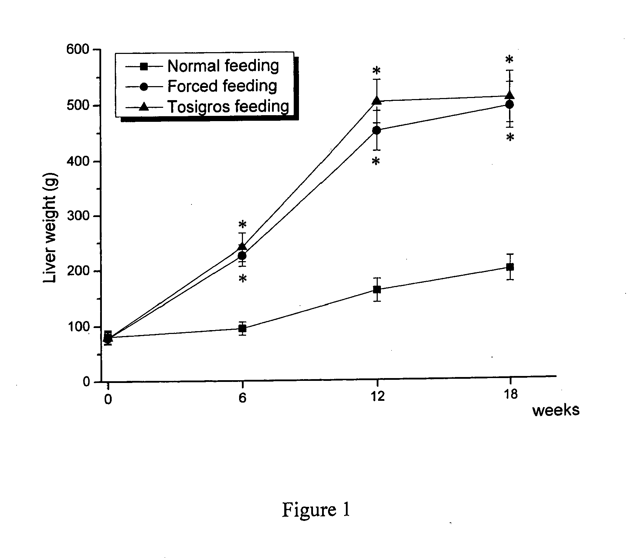

## Claims

1. A natural feed mixture for domesticated water birds, comprising a high carbohydrate containing basic component poor in methyl-donors, optionally a non-carbohydrate-based component of high caloric value and/or a carbohydrate or protein, amino acid mixture, fat, as well as phospholipid, cholesterol ester, and fatty acid-containing component, and natural flavouring as the main feed, **characterized in that** the main feed is supplemented with a component rich in carbohydrates, having hepatic insulin-sensitizing properties, as well as a feed supplement, comprising hypothalamic appetite-stimulating plant parts, natural substances blocking cholesterol synthesis in the liver, and an additive for protecting the formation of non-cholesterol-mevalonate products.

2. A natural feed supplement for domesticated water birds, comprising hypothalamic appetite-stimulating plant parts, natural substances blocking cholesterol synthesis in the liver, and an additive for protecting the formation of non-cholesterol-mevalonate products.

3. The feed mixture according to claim 1, **characterized in that** the high carbohydrate containing basic component poor in methyl-donors is corn.

4. The feed mixture according to claim 1, **characterized in that** the component rich in carbohydrates, having hepatic insulin-sensitizing properties, is sweet potato.

5. The feed mixture according to claim 1, **characterized in that** the non-carbohydrate-based component of high caloric value is pig fat.

6. The feed supplement according to claim 2, **characterized in that** the hypothalamic appetite-stimulating plant parts are fenugreek seed and/or seed juice.

7. The feed supplement according to claim 2, **characterized in that** the natural substances blocking cholesterol synthesis in the liver are bile acids.

8. The feed supplement according to claim 2, **characterized in that** the additive for protecting the formation of non-cholesterol-mevalonate products is farnesol oil.

9. The natural feed mixture according to claim 1, **characterized in that** it comprises 50 - 90 w% of corn, 50 - 90 w% of sweet potato, 0.1 - 10 w% of fenugreek seed, 0.1 -10 w% of a 1:20 - 20:1 mixture of lithocholic acid and ursodeoxycholic acid, 0.1 - 1 w% of fanresol oil, 1 - 10 w% of pig fat, and 0.1 - 2 w% of iodized NaCl.

10. The feed supplement according to claim 2, **characterized in that** it comprises 10-99 w% of fenugreek seed, 0.1 -10 w% of a 1:20 - 20:1 mixture of lithocholic acid and ursodeoxycholic acid, and 0.1 - 5 w% of farnesol oil.
